# EUROPÄISCHE PATENTSCHRIFT

(11) **EP 1 425 000 B1**
(45) Veröffentlichungstag und Bekanntmachung des Hinweises auf die Patenterteilung: **01.08.2012**
(21) Anmeldenummer: 02797584.6
(22) Anmeldetag: 17.08.2002
(51) Int. Cl.: A61K 31/10, A61P 3/04

(54) **VERWENDUNG VON MEHRFACH SUBSTITUIERTEN INDAN-1-OL-SYSTEMEN ZUR HERSTELLUNG VON MEDIKAMENTEN ZUR PROPHYLAXE ODER BEHANDLUNG VON OBESITAS**
USE OF POLYSUBSTITUTED INDAN-1-OL SYSTEMS FOR PRODUCING DRUGS USED IN THE PROPHYLAXIS OR TREATMENT OF OBESITY
UTILISATION DE SYSTEMES INDAN-1-OL POLYSUBSTITUES POUR LA PRODUCTION DE MEDICAMENTS SERVANT A LA PROPHYLAXIE OU AU TRAITEMENT DE L'OBESITE

(30) Priorität: 31.08.2001 DE 10142659
(43) Veröffentlichungstag der Anmeldung: 09.06.2004
(73) Patentinhaber: Sanofi-Aventis Deutschland GmbH, 65929 Frankfurt am Main (DE)
(72) Erfinder: JAEHNE, Gerhard, 65929 Frankfurt (DE); KRONE, Volker, 65719 Hofheim (DE); BICKEL, Martin, 61348 Bad Homburg (DE); GOSSEL, Matthias, 65719 Hofheim (DE)
(86) Internationale Anmeldenummer: PCT/EP2002/009200
(87) Internationale Veröffentlichungsnummer: WO 2003/020255

(56) Entgegenhaltungen:
- WO-A-01/02373
- WO-A-01/62746
- WO-A-97/20806
- FR-A- 2 345 146

## Beschreibung

Die Erfindung betrifft die Verwendung von mehrfach substituierten Indan-1-ol-Systemen sowie derer physiologisch verträgliche, Salze zur Herstellung von Medikamenten zur Prophylaxe oder Behandlung von Obesitas.

Aus WO 97/20806 sind cyclopentylsubstituierte Indan-1-ol-Derivate als entzündungshemmende Substanzen benannt.

In WO01/02373 werden Dihydrobenzodiazepine zur Behandlung von Dyslipidämie, Artheriosklerose und Diabetes beschrieben.

Der Erfindung lag die Aufgabe zugrunde, Verbindungen zur Verfügung zu stellen, die als Wirkstoffe zur Gewichtsreduktion bei Säugetieren eingesetzt werden können. Inbesonders sollen die Verbindungen eine therapeutisch verwertbare anorektische Wirkung entfalten.

Die Erfindung betrifft daher die Verwendung der Verbindungen der Formel I, 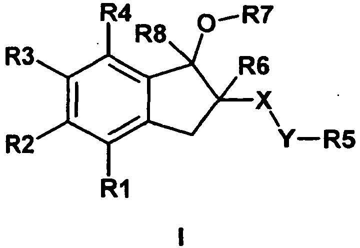 worin bedeuten
- R1, R2, R3, R4: unabhängig voneinander H, F, Cl, Br, J, CN, N₃, NO₂, OH, O(C₁-C₈)-Alkyl, O(C₃-C₈)-Cycloalkyl, O-CH₂-Phenyl, O-Phenyl, O-CO-(C₁-C₈)-Alkyl, O-CO-(C₃-C₈)-Cycloalkyl, wobei in den Alkylresten bis zu sieben Wasserstoffatome durch Fluor ersetzt sein können;
S(O)₀₋₂(C₁-C₈)-Alkyl, S(O)₀₋₂(C₃-C₈)-Cycloalkyl, wobei in den Alkylresten bis zu sieben Wasserstoffatome durch Fluor ersetzt sein können;
NH₂, NH-(C₁-C₈)-Alkyl, NH-(C₃-C₈)-Cycloalkyl, N[(C₁-C₈)-Alkyl]₂, N[(C₃-C₈)-Cycloalkyl]2, NH-CO-(C₁-C₈)-Alkyl, NH-CO-(C₃-C₈)-Cycloalkyl;
SO₃H; SO₂-NH₂, SO₂-NH-(C₁-C₈)-Alkyl, SO₂-NH-(C₃-C₈)-Cycloalkyl; SO₂-(C₁-C₆)-Alkyl;
NH-SO₂-NH₂; NH-SO₂-(C₁-C₈)-Alkyl, NH-SO₂-(C₃-C₈)-Cycloalkyl; O-CH₂-COOH, O-CH₂-CO-O(C₁-C₈)-Alkyl, COOH, COO(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂;
(C₁-C₈)-Alkyl, (C₃-C₈)-Cycloalkyl, (C₂-C₈)-Alkenyl, (C₂-C₈)-Alkinyl, wobei in den Alkyl-, Alkenyl- und Alkinylgruppen ein bis zu sieben Wasserstoffatome durch Fluor ersetzt sein können;
oder ein Wasserstoff durch OH, OC(O)CH₃, O-CH₂-Ph, NH₂, NH-CO-CH₃ oder N(COOCH₂Ph)₂ ersetzt sein kann;
Phenyl, 1- oder 2-Naphthyl,
5-Tetrazolyl, 1-[(C₁-C₆)-Alkyl]-5-Tetrazolyl, 2-[(C₁-C₆)-Alkyl]-5-Tetrazolyl;
1-Imidazolyl;
1-oder 4-[1,2,4]Triazolyl,
2- oder 3-Thienyl,
2- oder 3-Furyl,
2-, 3- oder 4-Pyridyl,
2-, 4- oder 5-Oxazolyl,
3-, 4- oder 5-Isoxazolyl,
2-, 4- oder 5-Thiazolyl,
3-, 4- oder 5-Isothiazolyl
wobei der Arylrest oder Heterocyclus bis zu zweifach mit
F, Cl, Br, CN,
OH, (C₁-C₄)-Alkyl, CF3, O-(C₁-C₄)-Alkyl,
S(O)₀₋₂(C₁-C₆)-Alkyl, NH₂, NH-SO₂-(C₁-C₄)-Alkyl;
COOH, CO-O-(C₁-C₄)-Alkyl, CO-NH₂ substituiert sein kann und in den Alkylgruppen ein bis sieben Wasserstoffatome durch Fluor ersetzt sein können; oder
- R2 und R3: bilden einen gemeinsam den Rest -O-CH₂-O-;
- X: S, SO, SO₂;
- Y: (CH₂)ₚ, wobei p gleich 0, 1, 2 oder 3 sein kann;
- R5: (C₁-C₁₈)-Alkyl, (C₃-C₈)-Cycloalkyl, wobei in den Alkylgruppen bis zu sieben Wasserstoffatome durch Fluor ersetzt sein können;
(CH₂)₁₋₆-COOH, (CH2)₁₋₆-COO-(C₁-C₆)-Alkyl, (CH₂)₁₋₆-CONH₂ sein kann; CH₂-CH(NHR10)-COR11, wobei R10 gleich H oder C(O)-(C₁-C₆)-Alkyl und R11 gleich OH, O-(C₁-C₆)-Alkyl oder NH₂ sein kann;
Phenyl, 1- oder 2-Naphthyl, Biphenyl, oder ein Heterocyclischer Rest, wobei die Ringe oder Ringsysteme jeweils bis zu dreifach substituiert sind mit
F, Cl, Br, J, CN,OH, O(C₁-C₈)-Alkyl, O(C₃-C₈)-Cycloalkyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₁-C₈)-Alkyl, NH-CO-(C₃-C₈)-Cycloalkyl, SO₃H; SO₂-NH₂, SO₂-NH-(C₁-C₈)-Alkyl, SO₂-NH-(C₃-C₈)-Cycloalkyl, NH-SO₂-NH₂; NH-SO₂-(C₁-C₈)-Alkyl, NH-SO₂-(C₃-C₈)-Cycloalkyl; O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂;
(C₁-C₈)-Alkyl, (C₃-C₈)-Cycloalkyl, wobei in den Alkylgruppen jeweils ein bis zu sieben Wasserstoffatome durch Fluor ersetzt sein können;
- R6: (CH₂)₀₋₆-R9, (CH₂)₀₋₆-COOH, (CH₂)₀₋₆-COO-(C₁-C₆)-Alkyl, (CH₂)₀₋₆-CONH₂, (CH₂)₀₋₆-CH(NHR15)-COR16, F, Cl, Br, CN, (C₁-C₁₈)-Alkyl, (C₃-C₄)-Cycloalkyl, (C₆-C₈)-Cycloalkyl, wobei in den Alkylresten oder Cycloalkylresten bis zu sieben Wasserstoffatome durch Fluor ersetzt sein können;
- R15: H, C(O)-(C₁-C₆)-Alkyl;
- R16: OH, O-(C₁-C₆)-Alkyl, NH₂;
- R7: (CH₂)₀₋₄-R12, H, (C₁-C₁₂)-Alkyl, (C₃-C₄)-Cycloalkyl, (C₆-C₈)-Cycloalkyl, COO(C₁-C₆)-Alkyl, COO(C₃-C₈)-Cycloalkyl, wobei in den Alkylresten oder Cycloalkylresten bis zu sieben Wasserstoffatome durch Fluor ersetzt sein können;
- R8: (CH₂)₀₋₄R14, (C₁-C₁₂)-Alkyl, (C₃-C₄)-Cycloalkyl, (C₆-C₈)-Cycloalkyl, wobei in den Alkyl- oder Cycloalkylresten bis zu sieben Wasserstoffatome durch Fluoratome ersetzt sein können;
- R9, R12, R14: unabhängig voneinander Phenyl, 1- oder 2-Naphthyl, Biphenyl, oder ein Heterocyclischer Rest, wobei die Ringe oder Ringsysteme jeweils bis zu dreifach substituiert sind mit
F, Cl, Br, J, CN,OH, O(C₁-C₈)-Alkyl, O(C₃-C₈)-Cycloalkyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₁-C₈)-Alkyl, NH-CO-(C₃-C₈)-Cycloalkyl, SO₃H; SO₂-NH₂, SO₂-NH-(C₁-C₈)-Alkyl, SO₂-NH-(C₃-C₈)-Cycloalkyl, NH-SO₂-NH₂; NH-SO₂-(C₁-C₈)-Alkyl, NH-SO₂-(C₃-C₈)-Cycloalkyl; O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂; (C₁-C₈)-Alkyl, (C₃-C₈)-Cycloalkyl, wobei in den Alkylgruppen jeweils ein bis zu sieben Wasserstoffatome durch Fluor ersetzt sein können;
sowie derer physiologisch verträglichen Salze zur Herstellung eines Medikaments zur Gewichtsreduktion von Säugetieren.

Die Erfindung bezieht sich auf Verwendung der Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R2, R3, R4, R5, R6, R7, R8, R9, R10, R11 und R12 können sowohl geradkettig wie verzweigt sein.

Unter Heterocyclus bzw. Heterocyclischer Rest werden Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist. Bevorzugte Hetercyclen bzw. Heterocyclische Reste sind:
Heteroaryl wie
Benzimidazolyl,
1-[(C₁-C₆)-Alkyl]-Benzimidazolyl,
Imidazolyl,
2- oder 3-Thienyl,
2- oder 3-Furyl,
Benzoxazolyl,
Benzthiazoyl, 2-, 3- oder 4-Pyridyl,
Pyrimidinyl,
4-, 5- oder 6-Pyridazin-2H-3-on-yl,
4-, 5- oder 6-Pyridazin-2-(C₁-C₈)-Alkyl-2H-3-on-yl,
4-, 5- oder 6-Pyridazin-2-benzyl-2H-3-on-yl,
3- oder 4-Pyridazinyl,
2-, 3-, 4- oder 8-Chinolinyl,
1-, 3- oder 4-Isochinolinyl,
1-Phthalazinyl,
3- oder 4-Cinnolinyl,
2- oder 4-Chinazolinyl,
2-Pyrazinyl,
2-Chinoxalinyl,
2-, 4- oder 5-oxazolyl,
3-, 4- oder 5-Isoxazolyl,
2-, 4- oder 5-Thiazolyl,
3-, 4- oder 5-Isothiazolyl,
1-[(C₁-C₆)-Alkyl]-2-, 4- oder 5-Imidazolyl,
3-, 4- oder 5-Pyrazolyl,
1-[(C₁-C₆)-Alkyl]-3-, 4- oder 5-Pyrazolyl,
1- oder 4-[1,2,4]Triazolyl,
4- oder 5-[1,2,3]Triazolyl,
1-[(C₁-C₆)-Alkyl]-4- oder 5-[1.2.3]Triazolyl,
3-, 4- oder 7-Indolyl,
N-[(C₁-C₆)-Alkyl]-3-, 4- oder 7-Indolyl
2-[(C₁-C₆)-Alkyl]-3(2H)-Indazolyl,
1-[(C₁-C₆)-Alkyl]-3(1H)-Indazolyl,
5-Tetrazolyl,
1-[(C₁-C₆)-Alkyl]-1H-Tetrazolyl,
2-[(C₁-C₆)-Alkyl]-2H-Tetrazolyl.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure ferner L-Ascorbinsäure, Salizylsäure, 1,2-Benzisothiazol-3(2H)-on und 6-Methyl-1,2,3-oxathiazin-4(3H)-on-2,2-dioxid. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Man kann Verbindungen der allgemeinen Formel I herstellen, indem nach folgenden Reaktionsschemata vorgegangen wird:

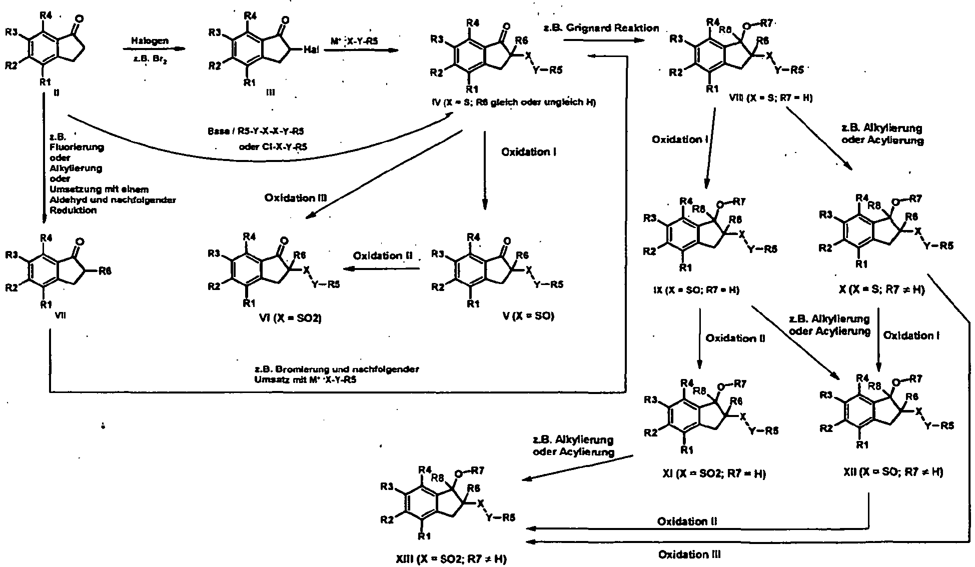

Dazu werden Verbindungen der allgemeinen Formel II, 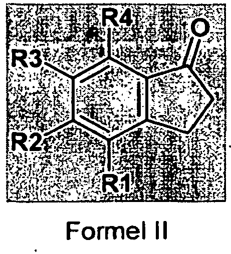 worin R1, R2, R3 und R4 die angegebene Bedeutung besitzen, mit einem Halogen wie z. B. Brom oder Chlor in eine Verbindung der Formel III überführt.
Die Verbindungen der Formel III werden weiter mit Metallsalzen von Thiolen der allgemeinen Formel H-X-Y-R5, wobei X gleich Schwefel ist und Y und R5 die angegebenen Bedeutungen besitzen, in Verbindungen der Formel IV mit X = S und R6 = H überführt. Diese Metallsalze können als solche eingesetzt werden oder in Lösung in situ aus dem Thiol und einer Base wie z. B. wässrigem Natriumhydroxid erzeugt werden.
Andererseits kann man Verbindungen der Formel IV mit X = S und R6 = H durch Umsatz von Verbindungen der Formel II mit einer Base wie z.B. Lithiumdiisopropylamid in z.B. Tetrahydrofuran und einem Disulfid der allgemeinen Formel R5-Y-X-X-Y-R5 erhalten, worin R5 und Y die angegebenen Bedeutungen besitzen und X = S ist; alternativ kann statt des Disulfids auch ein Sulfenylchlorid der allgemeinen Formel Cl-X-Y-R5, wobei X = S ist und Y und R5 die angegebenen Bedeutungen haben, eingesetzt werden (siehe z.B. D. Seebach et al.; Chem. Ber. 109, 1601-1616 (1976)).

Verbindungen der Formel IV, worin X = S und R6 ungleich Wasserstoff ist, lassen sich z.B. folgendermassen gewinnen: Verbindungen der Formel II werden z.B. einer Fluorierung, Alkylierung oder einer Kondensation mit einem Aldehyd und nachfolgender Reduktion unterzogen, so daß man zu Verbindungen der Formel VII gelangt, welche ihrerseits z.B. nach erfolgter Bromierung mit Verbindungen der Formel M^{+ -}X-Y-R5, wobei X = S ist und Y und R5 die oben beschriebenen Bedeutungen haben, zu Verbindungen der Formel IV mit X = S und R6 ungleich Wasserstoff umgesetzt werden können.

Verbindungen der Formel V, worin X = SO und R6 ungleich Wasserstoff ist, können z.B. durch selektive Oxidation der Verbindung der Formel IV, worin X = S ist, mit einem Äquivalent Peroxytrifluoressigsäure (C. G. Venier et al.; J. Org. Chem. 47, 3773 (1982)) hergestellt werden. Die Herstellung der Sulfoxide aus den Sulfiden kann auch mittels Mangandioxid oder Chromsäure erfolgen (D. Edwards et al.; J. Chem. Soc. 1954, 3272). Für diese Oxidation ist weiterhin auch Wasserstoffperoxid in Essigsäureanhydrid geeignet (A. V. Sviridova et al.; J. Org. Chem (Russ), English Transl.; 7, 2577 (1971).

Verbindungen der Formel VI, worin X = SO2 und R6 ungleich Wasserstoff ist, können durch Oxidation mit z.B. 2KHSO₅ x KHSO₄ x K₂SO₄ (Oxone) entweder aus Verbindungen der Formel IV, worin X = S und R6 ungleich Wasserstoff ist, oder aus Verbindungen der Formel V, worin X = SO und R6 ungleich Wasserstoff ist, gewonnen werden (siehe z.B. M. Hudlický, Oxidations in Organic Chemistry, ACS Monograph 186, American Chemical Society, Washington, DC, 1990).

Verbindungen der Formel VIII, worin R8 ungleich Wasserstoff, R7 gleich Wasserstoff und X = S ist, lassen sich z.B. durch Reaktion von Verbindungen der Formel IV mit einem Grignard Reagenz gewinnen. Schrittweise Oxidationen und Alkylierungs- oder Acylierungsreaktionen erlauben den Zugang zu Verbindungen der Formeln IX bis XIII. Ein weiterer Zugang zu solchen Verbindungen ist durch den Einsatz von Verbindungen der Formeln V oder VI in die Grignard Reaktion möglich.

Zur Salzbildung kommen als anorganische Säuren beispielsweise in Betracht: Halogenwasserstoffsäuren wie Chlorwasserstoffsäure und Bromwasserstoffsäure, sowie Schwefelsäure, Phosphorsäure und Amidosulfonsäure.

Als organische Säuren zur Salzbildung seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

**Tabelle 1: Beispiele**

| 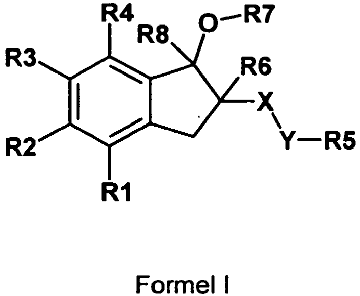 | | | | | | | | | | | |
|---|---|---|---|---|---|---|---|---|---|---|---|
| Bsp. | R1 | R2 | R3 | R4 | X | Y | R5 | R6 | R7 | R8 | Fp. [°C] |
| 1 | H | Cl | H | H | SO₂ | - | CH₃ | F | H | CH₃ | 148 |
| | | | | | | | | | | | |
| | | | | | | | | | | | [MH+] |
| 2 | H | Cl | H | H | SO₂ | - | CH₃ | F | H | CF₃ | 333.2 |

Die Verbindungen der Formel I zeichnen sich durch günstige Wirkungen auf den Fettstoffwechsel aus, insbesondere sind sie zur Gewichtreduktion und nach erfolgter Gewichtsreduktion zum Erhalt eines reduzierten Gewichtes bei Säugetieren und als Anorektika geeignet. Die Verbindungen zeichnen sich durch ihre geringe Toxizität und ihre geringen Nebenwirkungen aus. Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas
Bei einem weiteren Aspekt der Erfindung können die Verbindungen der Formel I in Kombination mit einer oder mehreren weiteren pharmakologisch wirksamen Substanzen verabreicht werden, die beispielsweise ausgewählt sind aus Antidiabetika, Antiadiposita, blutdrucksenkenden Wirkstoffen, Lipidsenkern und Wirkstoffen zur Behandlung und/oder Prävention von Komplikationen, die von Diabetes verursacht werden oder mit Diabetes assoziiert sind.

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer weiteren Ausführungsform werden die vorliegenden Verbindungen in Kombination mit einem Sulphonylharnstoff wie z.B. Tolbutamid, Glibenclamid, Glimepirid, Glipizid, Gliquidon, Glisoxepid, Glibornurid oder Gliclazid verabreicht.

Bei einer anderen Ausführungsform werden die vorliegenden Verbindungen in Kombination mit einem Biguanid wie z.B. Metformin verabreicht.

Bei wieder einer anderen Ausführungsform werden die vorliegenden Verbindungen in Kombination mit einem Meglitinid wie z.B. Repaglinid verabreicht.

Bei noch einer weiteren Ausführungsform werden die vorliegenden Verbindungen in Kombination mit einem Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei noch einer weiteren Ausführungsform werden die vorliegenden Verbindungen in Kombination mit einem Monoamine Oxidase Inhibitor, wie z.B. in WO 01/12176 offenbart, verabreicht. Besonders geeignet sind dabei [3(S),3a(S)]-3-methoxymethyl-7-[4,4,4-trifluorobutoxy]-3,3a,4,5-tetrahydro-1H-oxazolo[3,4-a]quinolin-1-on, (R)-5-(methoxymethyl)-3-[6-(4,4,4-trifluorobutoxy)benzofuran-3-yl]oxazolidin-2-one oder (R)-5-(methoxymethyl)-3-[6-cyclopropylmethoxybenzofuran-3-yl]oxazolidin-2-one.

Bei einer weiteren Ausführungsform werden die vorliegenden Verbindungen in Kombination mit einem α-Glukosidase-Inhibitor wie z.B. Miglitol oder Acarbose verabreicht.

Bei noch einer weiteren Ausführungsform werden die vorliegenden Verbindungen in Kombination mit einem hCNTF (human ciliary neurotrophic factor) oder Derivaten davon wie z.B. CNTF_{AX15} oder modifiziertes CNTF_{AX15}, wie z.B. in Lambert et al., PNAS 98, 4652-4657 offenbart, verabreicht.

Bei einer anderen Ausführungsform werden die vorliegenden Verbindungen in Kombination mit einem Wirkstoff verabreicht, der auf den ATP-abhängigen Kaliumkanal der Betazellen wirkt, wie z.B. Tolbutamid, Glibenclamid, Glimepirid, Glipizid, Gliclazid oder Repaglinid.

Bei noch einer anderen Ausführungsform werden die vorliegenden Verbindungen in Kombination mit einem antihyperlipidämischen Wirkstoff oder einem antilipidämischen Wirkstoff wie z.B. Cholestyramin, Colestipol, Clofibrat, Gemfibrozil, Lovastatin, Pravastatin, Simvastatin, Atorvastatin, Cerivastatin, Fluvastatin, Probucol, Ezetimibe oder Dextrothyroxin verabreicht.

Bei einer weiteren Ausführungsform werden die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Weiterhin können die erfindungsgemäßen Verbindungen in Kombination mit einem oder mehreren Antiadiposita oder appetitregulierenden Wirkstoffen verabreicht werden.

Solche Wirkstoffe können ausgewählt werden aus der Gruppe bestehend aus CART-Agonisten, NPY-Antagonisten, Melanocortin 3 oder 4 (MC3 oder MC4) - Agonisten, Melanin-konzentrierendes Hormon (MCH) - Antagonisten, Orexin-Antagonisten, H3-Agonisten, TNF-Agonisten, CRF-Agonisten, CRF BP-Antagonisten, Urocortin-Agonisten, β3-Adrenoceptor Agonisten, CCK-Agonisten, Serotonin-Wiederaufnahme-Inhibitoren, gemischte Sertonin- und Noradrenalin-Wiederaufnahme-Inhibitoren, 5HT-Modulatoren, Bombesin-Agonisten, Galanin-Antagonisten, Glucocorticoid Rezeptor Modulatoren, Wachstumshormon, Wachstumshormon freisetzende Verbindungen, TRH-Agonisten, Modulatoren der Entkopplungsproteine 2 oder 3, Leptin Rezeptor Agonisten, Leptinmimetika, Dopamin-Agonisten (Bromocriptin, Doprexin), Lipase/Amylase-Inhibitoren, Antagonisten des Cannabinoid Rezeptors 1, Modulatoren des die Acylierung stimulierendes Protein (ASP), PPAR-Modulatoren, RXR-Modulatoren oder TR-β-Agonisten.

Bei einer Ausführungsform der Erfindung ist das Antiadipositum Leptin oder modifiziertes Leptin.

Bei einer anderen Ausführungsform ist das Antiadipositum Dexamphetamin oder Amphetamin.

Bei einer anderen Ausführungsform ist das Antiadipositum Fenfluramin oder Dexfenfluramin.

Bei noch einer anderen Ausführungsform ist das Antiadipositum Sibutramin oder die mono- und bisdemethylierten Wirkmetabolite von Sibutramin.

Bei einer weiteren Ausführungsform ist das Antiadipositum Orlistat.

Bei einer anderen Ausführungsform ist das Antiadipositum Mazindol, Diethylpropion oder Phentermin.

Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propanolol und Metoprolol, ACE (Angiotensin Converting Enzym)-Hemmer wie z.B. Benazepril, Captopril, Enalapril, Fosinopril, Lisinopril, Quinapril und Rampril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.

Die folgenden Zubereitungen dienen zur Erläuterung der Erfindung ohne diese jedoch einzuschränken.

### Beispiel A

Gelatineweichkapseln, enthaltend 100 mg Wirkstoff pro Kapsel:

| | pro Kapsel |
|---|---|
| Wirkstoff aus Kokosfett fraktioniertes | 100 mg |
| Triclycerid-Gemisch | 400 mg |
| Kapselinhalt | 500 mg |

### Beispiel B

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel C

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

### Beispiel D

Tabletten, enthaltend 40 mg Wirkstoff pro Tablette:

| | pro Tablette |
|---|---|
| Lactose | 600 mg |
| Maisstärke | 300 mg |
| lösliche Stärke | 20 mg |
| Magnesiumstearat | 40 mg |
| | 1000 mg |

### Beispiel E

Dragees, enthaltend 50 mg Wirkstoff pro Dragees:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

### Beispiel F

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

### Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

Die Wirksamkeit der Verbindungen der Formel I wurde wie folgt getestet:

### Biologisches Prüfmodell:

Die Prüfung der anorektischen Wirkung erfolgte an weiblichen NMRI Mäusen. Nach 24stündigem Futterentzug wurde das Testpräparat intraperitoneal (ip) oder über eine Schlundsonde (po) verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

**Tabelle 2: Anorektische Wirkung, gemessen als Reduktion des kumulierten Milchkonsums behandelter im Vergleich zu dem unbehandelter Tiere.**

| Verbindung/Beispiel | Dosis [mg/kg] | Anzahl der Tiere / Kumulierter Milchkonsum der behandelten Tiere N / [ml] | Anzahl der Tiere / Kumulierter Milchkonsum der unbehandelten Kontrolltiere N / [ml] | Reduktion des kumulierten Milchkonsums in % der Kontrolle |
|---|---|---|---|---|
| 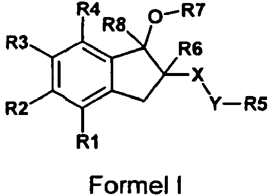 | | | | |
| | | | | |
| Beispiel 1 | 30 (ip) | 4 / 2,88 | 5 / 3,86 | 25 |

Aus der Tabelle ist abzulesen, daß die Verbindungen der Formel I eine gute anorektische Wirkung zeigen.

Nachfolgend wird die Herstellung einiger Beispiele detailliert beschrieben, die übrigen Verbindungen der Formel I wurden analog erhalten:

### Beispiel 1:

### 5-Chlor-2-fluor-2-methansulfonyl-1-methyl-indan1-ol:

### 1. 5-Chlor-2-methylsulfanyl-indan-1-on:

0.98 g (4 mmol) 2-Brom-5-chlor-indan-1-on und 0.42 g (6 mmol) Natriumthiomethylat werden in 5 ml Ethanol suspendiert, 30 Minuten im Ultraschallbad behandelt und anschließend 90 Minuten bei Raumtemperatur gerührt. Die Reaktionsmischung wird im Vakuum eingeengt und über Kieselgel mit Toluol/Essigsäureethylester 10/1 chromatographiert. Die Eluate werden im Vakuum eingeengt und liefern 0.63 g 5-Chlor-2-methylsulfanyl-indan-1-on mit dem Schmelzpunkt 90°C.

### 2. 5-Chlor-2-methansulfonyl-indan-1-on:

0.5 g (2.35 mmol) 5-Chlor-2-methylsulfanyl-indan-1-on werden in 10 ml Methanol gelöst; bei 0°C wird eine Lösung aus 4.33 g (7.05 mmol) 2KHSO₅ x KHSO₄ x K₂SO₄ in 10 ml Wasser zugetropft. Die Mischung wird 5 h bei Raumtemperatur gerührt; das Methanol wird abdestilliert und der wässrige Rückstand wird mit Dichlormethan extrahiert. Die organische Phase wird abgetrennt, über MgSO₄ getrocknet, filtriert und im Vakuum eingeengt. Man erhält 0.5 g 5-Chlor-2-methansulfonyl-indan-1-on mit dem Schmelzpunkt 197°C.

### 3. 5-Chlor-2-fluor-2-methansulfonyl-indan-1-on:

0.734 g (3 mmol) 5-Chlor-2-methansulfonyl-indan-1-on und 1.77 g (5 mmol) N-Fluor-N'-(chlormethyl)triethylendiamin bis(tetrafluorborat) werden in einer Mischung aus 2.5 ml Wasser und 7.5 ml Acetonitril suspendiert und 4 h unter Rückfluß gerührt. Die Reaktionsmischung wird abgekühlt, im Vakuum eingeengt und über Kieselgel mit Dichlormethan als Laufmittel chromatographisch gereinigt. Man erhält 5-Chlor-2-fluor-2-methansulfonyl-indan-1-on mit dem Schmelzpunkt 150°C.

### 4. 5-Chlor-2-fluor-2-methansulfonyl-1-methyl-indan1-ol:

260 mg (1 mmol) 5-Chlor-2-fluor-2-methansulfonyl-indan-1-on werden bei Raumtemperatur in 10 ml trockenem Tetrahydrofuran gelöst und mit 0.33 ml einer 3 molaren Lösung von Methylmagnesiumbromid in Diethylether tropfenweise unter Rühren versetzt.. Die Reaktionsmischung wird 3 h bei 50°C gerührt. Danach werden weitere 0.33 ml der Methylmagnesiumbromid-Lösung zugegeben und es wird eine weitere Stunde bei Raumtemperatur gerührt. Nach beendeter Reaktion wird die Reaktionsmischung mit Essigsäureethylester verdünnt und nacheinander mit ges. Ammoniumchlorid-Lösung, ges. Natriumhydrogensulfitlösung, ges. Natriumhydrogencarbonat-Lösung und ges. Kochsalz-Lösung ausgeschüttelt. Die organische Phase wird über Magnesiumsulfat getrocknet, filtriert und im Vakuum eingeengt. Nach chromatographischer Reinigung über Kieselgel (n-Heptan/Essigsäureethylester - 60/40) wird 5-Chlor-2-fluor-2-methansulfonyl-1-methyl-indan1-ol mit dem Schmelzpunkt 148°C erhalten.

### Beispiel 2:

### 5-Chlor-2-fluor-2-methansulfonyl-1-trifluormethyl-indan-1-ol

Setzt man 5-Chlor-2-fluor-2-methansulfonyl-indan-1-on statt mit Methylmagnesiumbromid mit Trifluormethyltrimethylsilan und Tetrabutylammoniumfluorid in Tetrahydrofuran um, so wird 5-Chlor-2-fluor-2-methansulfonyl-1-trifluormethyl-indan-1-ol mit dem Molekulargewicht 332,7 (C₁₁H₉CIF₄SO₃); MS (ESI): 333,20 (MH⁺) erhalten.

## Patentansprüche

1. Verwendung der Verbindungen der Formel I, 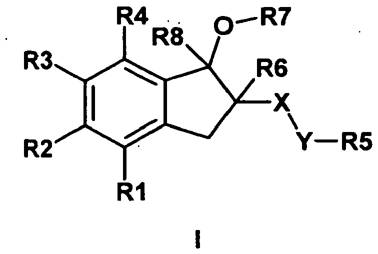 worin bedeuten
R1, R2, R3, R4 unabhängig voneinander H; F, Cl, Br, J, CN, N₃, NO₂, OH, O(C₁-C₈)-Alkyl, O(C₃-C₈)-Cycloalkyl, O-CH₂-Phenyl, O-Phenyl, O-CO-(C₁-C₈)-Alkyl, O-CO-(C₃-C₈)-Cycloalkyl, wobei in den Alkylresten bis zu sieben Wasserstoffatome durch Fluor ersetzt sein können;
S(O) ₀₋₂(C₁-C₈)-Alkyl, S(O)₀₋₂(C₃-C₈)-Cycloalkyl, wobei in den Alkylresten bis zu sieben Wasserstoffatome durch Fluor ersetzt sein können;
NH₂, NH-(C₁-C₈)-Alkyl, NH-(C₃-C₈)-Cycloalkyl, N[(C₁-C₈)-Alkyl]₂, N[(C₃-C₈)-Cycloalkyl]2, NH-CO-(C₁-C₈)-Alkyl, NH-CO-(C₃-C₈)-Cycloalkyl;
SO₃H; SO₂-NH₂, SO₂-NH-(C₁-C₈)-Alkyl, SO₂-NH-(C₃-C₈)-Cycloalkyl; SO₂-(C₁-C₆)-Alkyl;
NH-SO₂-NH₂; NH-SO₂-(C₁-C₈)-Alkyl, NH-SO₂-(C₃-C₈)-Cycloalkyl; O-CH₂-COOH, O-CH₂-CO-O(C₁-C₈)-Alkyl, COOH, COO(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂;
(C₁-C₈)-Alkyl, (C₃-C₈)-Cycloalkyl, (C₂-C₈)-Alkenyl, (C₂-C₈)-Alkinyl, wobei in den Alkyl-, Alkenyl- und Alkinylgruppen ein bis zu sieben Wasserstoffatome durch Fluor ersetzt sein können;
oder ein Wasserstoff durch OH, OC(O)CH₃, O-CH₂-Ph, NH₂, NH-CO-CH₃ oder N(COOCH₂Ph)₂ ersetzt sein kann;
Phenyl, 1- oder 2-Naphthyl,
5-Tetrazolyl, 1-[(C₁-C₆)-Alkyll-5-Tetrazolyl, 2-[(C₁-C₆)-Alkyl]-5-Tetrazolyl;
1-Imidazolyl;
1-oder 4-[1,2,4]Triazolyl,
2- oder 3-Thienyl,
2- oder 3-Furyl,
2-, 3- oder 4-Pyridyl,
2-, 4- oder 5-Oxazolyl,
3-, 4- oder 5-Isoxazolyl,
2-, 4- oder 5-Thiazolyl,
3-, 4- oder 5-Isothiazolyl
wobei der Arylrest oder Heterocyclus bis zu zweifach mit
F, Cl, Br, CN,
OH, (C₁-C₄)-Alkyl, CF3, O=(C₁-C₄)-Alkyl,
S(O)₀₋₂(C₁-C₆)-Alkyl, NH₂, NH-SO₂-(C₁-C₄)-Alkyl;
COOH, CO-O-(C₁-C₄)-Alkyl, CO-NH₂ substituiert sein kann und in den Alkylgruppen ein bis sieben Wasserstoffatome durch Fluor ersetzt sein können; oder
R2 und R3 bilden einen gemeinsam den Rest -O-CH₂-O-;
X S, SO, SO₂;
Y (CH₂)ₚ, wobei p gleich 0, 1, 2 oder 3 sein kann;
R5 (C₁-C₁₈)-Alkyl, (C₃-C₈)-Cycloalkyl,
wobei in den Alkylgruppen bis zu sieben Wasserstoffatome durch Fluor ersetzt sein können;
(CH₂)₁₋₆-COOH, (CH₂)₁₋₆-COO-(C₁-C₆)-Alkyl, (CH₂)₁₋₆-CONH₂ sein kann;
CH₂-CH(NHR10)-COR11, wobei R10 gleich H oder C(O)-(C₁-C₆)-Alkyl und R11 gleich OH, O-(C₁-C₆)-Alkyl oder NH₂ sein kann;
Phenyl, 1- oder 2-Naphthyl, Biphenyl, oder ein Heterocyclischer Rest, wobei die Ringe oder Ringsysteme jeweils bis zu dreifach substituiert sind mit
F, Cl, Br, J, CN,OH, O(C₁-C₈)-Alkyl, O(C₃-C₈)-Cycloalkyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₁-C₈)-Alkyl, NH-CO-(C₃-C₈)-Cycloalkyl, SO₃H; SO₂-NH₂, SO₂-NH-(C₁-C₈)-Alkyl, SO₂-NH-(C₃-C₈)-Cycloalkyl, NH-SO₂-NH₂; NH-SO₂-(C₁-C₈)-Alkyl, NH-SO₂-(C₃-C₈)-Cycloalkyl; O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂;
(C₁-C₈)-Alkyl, (C₃-C₈)-Cycloalkyl, wobei in den Alkylgruppen jeweils ein bis zu sieben Wasserstoffatome durch Fluor ersetzt sein können;
R6 (CH₂)₀₋₆-R9, (CH₂)₀₋₆-COOH, (CH₂)₀₋₆-COO-(C₁-C₆)-Alkyl, (CH₂)₀₋₆-CONH₂, (CH₂)₀₋₆-CH(NHR15)-COR16, F, Cl, Br, CN, (C₁-C₁₈)-Alkyl, (C₃-C₄)-Cycloalkyl, (C₆-C₈)-Cycloalkyl, wobei in den Alkylresten oder Cycloalkylresten bis zu sieben Wasserstoffatome durch Fluor ersetzt sein können;
R15 H, C(O)-(C₁-C₆)-Alkyl;
R16 OH, O-(C₁-C₆)-Alkyl, NH₂;
R7 (CH₂)₀₋₄-R12, H, (C₁-C₁₂)-Alkyl, (C₃-C₄)-Cycloalkyl, (C₆-C₈)-Cycloalkyl, COO(C₁-C₆)-Alkyl, COO(C₃-C₈)-Cycloalkyl, wobei in den Alkylresten oder Cycloalkylresten bis zu sieben Wasserstoffatome durch Fluor ersetzt sein können;
R8 (CH₂)₀₋₄R14, (C₁-C₁₂)-Alkyl, (C₃-C₄)-Cycloalkyl, (C₆-C₈)-Cycloalkyl, wobei in den Alkyl- oder Cycloalkylresten bis zu sieben Wasserstoffatome durch Fluoratome ersetzt sein können;
R9, R12, R14 unabhängig voneinander
Phenyl, 1- oder 2-Naphthyl, Biphenyl, oder ein Heterocyclischer Rest, wobei die Ringe oder Ringsysteme jeweils bis zu dreifach substituiert sind mit
F, Cl, Br, J, CN,OH, O(C₁-C₈)-Alkyl, O(C₃-C₈)-Cycloalkyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₁-C₈)-Alkyl, NH-CO-(C₃-C₈)-Cycloalkyl, SO₃H; SO₂-NH₂, SO₂-NH-(C₁-C₈)-Alkyl, SO₂-NH-(C₃-C₈)-Cycloalkyl, NH-SO₂-NH₂; NH-SO₂-(C₁-C₈)-Alkyl, NH-SO₂-(C₃-C₈)-Cycloalkyl; O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂;
(C₁-C₈)-Alkyl, (C₃-C₈)-Cycloalkyl, wobei in den Alkylgruppen jeweils ein bis zu sieben Wasserstoffatome durch Fluor ersetzt sein können;
sowie derer physiologisch verträglichen Salze zur Herstellung eines Medikaments zur Gewichtsreduktion von Säugetieren.

2. Verwendung gemäß Anspruch 1 zur Herstellung eines Medikaments zur Prophylaxe oder Behandlung von Obesitas.

3. Verwendung gemäß Anspruch 1 in Kombination mit einem oder mehren gewichtsreduzierenden Wirkstoffen zur Herstellung eines Medikaments zur Gewichtsreduktion von Säugetieren.

4. Verwendung gemäß Anspruch 1 in Kombination mit einem oder mehren gewichtsreduzierenden Wirkstoffen zur Herstellung eines Medikaments zur Prophylaxe oder Behandlung von Obesitas.

5. Verwendung gemäß Anspruch 1 in Kombination mit Cathine, Phenylpropanolamine, Amfepramone, Mefenorex, Ephedrine, Leptin, Dexamphetamine, Amphetamine, Fenfluramine, Dexfenfluramine, Sibutramine, Orlistat, Mazindol oder Phentermine oder deren Salze zur Herstellung eines Medikaments zur Gewichtsreduktion von Säugetieren.

6. Verwendung gemäß Anspruch 1 in Kombination mit Cathine, Phenylpropanolamine, Amfepramon, Mefenorex, Ephedrine, Leptin, Dexamphetamine, Amphetamine, Fenfluramine, Dexfenfluramine, Sibutramine, Orlistat, Mazindol oder Phentermine oder deren Salze zur Herstellung eines Medikaments zur Prophylaxe oder Behandlung von Obesitas.

## Claims

1. The use of the compounds of the formula I, 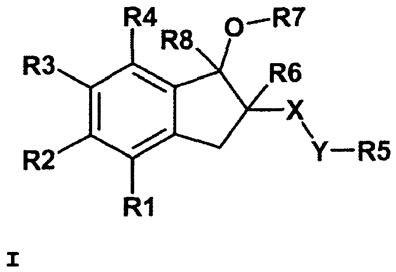 in which
R1, R2, R3, R4 independently of one another are H; F, Cl, Br, I, CN, N₃, NO₂, OH, O(C₁-C₈)-alkyl, O(C₃-C₈)-cycloalkyl, O-CH₂-phenyl, O-phenyl, O-CO-(C₁-C₈)-alkyl, O-CO-(C₃-C₈)-cycloalkyl, where in the alkyl radicals up to seven hydrogen atoms may be replaced by fluorine; S(O)₀₋₂(C₁-C₈) -alkyl, S(O)₀₋₂(C₃-C₈) -cycloalkyl, where in the alkyl radicals up to seven hydrogen atoms may be replaced by fluorine; NH₂, NH-(C₁-C₈)-alkyl, NH-(C₃-C₈)-cycloalkyl, N[(C₁-C₈)-alkyl]₂, N[(C₃-C₈)-cycloalkyl]₂, NH-CO-(C₁-C₈)-alkyl, NH-CO-(C₃-C₈)-cycloalkyl ; SO₃H; SO₂-NH₂, SO₂-NH-(C₁-C₈) -alkyl, SO₂-NH-(C₃-C₈)-cycloalkyl; SO₂-(C₁-C₆)-alkyl ; NH-SO₂-NH₂; NH-SO₂-(C₁-C₈)-alkyl, NH-SO₂-(C₃-C₈) - cycloalkyl; O-CH₂-COOH, O-CH₂-CO-O(C₁-C₈)-alkyl, COOH, COO(C₁-C₈)-alkyl, CO-O-(C₃-C₈)-cycloalkyl, CO-NH₂, CO-NH(C₁-C₈)-alkyl, CO-N[(C₁-C₈-alkyl]₂; (C₁-C₈) -alkyl, (C₃-C₈)cycloalkyl, (C₂-C₈-alkenyl, (C₂-C₈)-alkynyl, where in the alkyl, alkenyl and alkynyl groups one to seven hydrogen atoms may be replaced by fluorine; or one hydrogen may be replaced by OH, OC(O)CH₃, O-CH₂-Ph, NH₂, NH-CO-CH₃ or N(COOCH₂Ph)₂; phenyl, 1- or 2-naphthyl, 5-tetrazolyl, 1-[(C₁-C₆)-alkyl]-5-tetrazolyl, 2-[(C₁-C₆)-alkyl]-5-tetrazolyl,
1-imidazolyl,
1- or 4-[1,2,4]-triazolyl,
2- or 3-thienyl,
2- or 3-furyl,
2-, 3- or 4-pyridyl, 2-, 4- or 5-oxazolyl,
3-, 4- or 5-isoxazolyl, 2-, 4- or 5-thiazolyl,
3-, 4- or 5-isothiazolyl,
where the aryl radical or heterocycle may be substituted up to two times by F, Cl, Br, CN,
OH, (C₁-C₄)-alkyl, CF₃, O-(C₁-C₄)-alkyl, S(O)₀₋₂(C₁-C₆)-alkyl, NH₂, NH-SO₂-(C₁-C₄)-alkyl; COOH, CO-O-(C₁-C₄)-alkyl, CO-NH₂ and where in the alkyl groups one to seven hydrogen atoms may be replaced by fluorine; or
R2 and R3 together form the radical -O-CH₂-O-;
X is S, SO, SO₂;
Y is (CH₂)ₚ, where p may be 0, 1, 2 or 3;
R5 is (C₁-C₁₈)-alkyl, (C₃-C₈)-cycloalkyl, where in the alkyl groups up to seven hydrogen atoms may be replaced by fluorine;
(CH₂)₁₋₆-COOH, (CH₂)₁₋₆-COO- (C₁-C₆) -alkyl, (CH₂)₁₋₆-CONH₂;
CH₂-CH(NHR10)-COR11, where R10 may be H or C(O)-(C₁-C₆)-alkyl and R11 may be OH, O-(C₁-C₆)-alkyl or NH₂;
phenyl, 1- or 2-naphthyl, biphenyl or a heterocyclic radical, where the rings or ring systems are in each case substituted up to three times by
F, Cl, Br, I, CN, OH, O(C₁-C₈)-alkyl, O(C₃-C₈)-cycloalkyl, O-CO- (C₁-C₈)-alkyl , O-CO-(C₃-C₈)-cycloalkyl, S(O)₀₋₂(C₁-C₈)-alkyl, S(O)₀₋₂(C₃-C₈)-cycloalkyl, NH₂, NH-(C₁-C₈)-alkyl, NH-(C₃-C₈)-cycloalkyl, N[(C₁-C₈)-alkyl]₂, N[(C₃-C₈)-cycloalkyl]₂, NH-CO-(C₁-C₈)-alkyl, NH-CO-(C₃-C₈)-cycloalkyl, SO₃H; SO₂-NH₂, SO₂-NH-(C₁-C₈)-alkyl, SO₂-NH-(C₃-C₈)-cycloalkyl, NH-SO₂-NH₂; NH-SO₂- (C₁-C₈)-alkyl, NH-SO₂- (C₃-C₈)-cycloalkyl; O-CH₂-COOH, O-CH₂-CO-O(C₁-C₈)-alkyl, COOH, COO(C₁-C₈)-alkyl, CO-O-(C₃-C₈)-cycloalkyl, CO-NH₂, CO-NH(C₁-C₈)-alkyl, CO-N[(C₁-C₈)-alkyl]₂; (C₁-C₈)-alkyl, (C₃-C₈)-cycloalkyl, where in the alkyl groups in each case one to seven hydrogen atoms may be replaced by fluorine;
R6 is (CH₂)₀₋₆-R9, (CH₂)₀₋₆-COOH, (CH₂)₀₋₆-COO-(C₁-C₆)-alkyl, (CH₂)₀₋₆-CONH₂, (CH₂)₀₋₆-CH(NHR15)-COR16, F, Cl, Br, CN, (C₁-C₁₈)-alkyl, (C₃-C₄)-cycloalkyl, (C₆-C₈)-cycloalkyl, where in the alkyl radicals or cycloalkyl radicals up to seven hydrogen atoms may be replaced by fluorine;
R15 is H, C(O)-(C₁-C₆)-alkyl;
R16 is OH, O-(C₁-C₆)-alkyl, NH₂;
R7 is (CH₂)₀₋₄-R12, H, (C₁-C₁₂)-alkyl, (C₃-C₄)-cycloalkyl, (C₆-C₈)-cycloalkyl, COO(C₁-C₆)-alkyl, COO(C₃-C₈)-cycloalkyl, where in the alkyl radicals or cycloalkyl radicals up to seven hydrogen atoms may be replaced by fluorine;
R8 is (CH₂)₀₋₄-R14, (C₁-C₁₂)-alkyl, (C₃-C₄)-cycloalkyl, (C₆-C₈)-cycloalkyl, where in the alkyl or cycloalkyl radicals up to seven hydrogen atoms may be replaced by fluorine atoms;
R9, R12, R14 independently of one another are phenyl, 1- or 2-naphthyl, biphenyl, or a heterocyclic radical, where the rings or ring systems are in each case substituted up to three times by
F, Cl, Br, I, CN, OH, O(C₁-C₈)-alkyl, O(C₃-C₈)-cycloalkyl , O-CO- (C₁-C₈)-alkyl, O-CO- (C₃-C₈)-cycloalkyl, S(O)₀₋₂(C₁-C₈)-alkyl, S(O)₀₋₂(C₃-C₈)-cycloalkyl, NH₂, NH-(C₁-C₈)-alkyl, NH-(C₃-C₈)-cycloalkyl, N[(C₁-C₈)-alkyl]₂, N[(C₃-C₈)-cycloalkyl]₂, NH-CO-(C₁-C₈) -alkyl, NH-CO- (C₃-C₈)-cycloalkyl, SO₃H; SO₂-NH₂, SO₂-NH-(C₁-C₈)-alkyl, SO₂-NH- (C₃-C₈)-cycloalkyl, NH-SO₂-NH₂; NH-SO₂-(C₁-C₈)-alkyl, NH-SO₂-(C₃-C₈)-cycloalkyl; O-CH₂-COOH, O-CH₂-CO-O(C₁-C₈)-alkyl, COOH, COO (C₁-C₈)-alkyl, CO-O-(C₃-C₈)-cycloalkyl, CO-NH₂, CO-NH(C₁-C₈)-alkyl, CO-N[(C₁-C₈)-alkyl]₂; (C₁-C₈)-alkyl, (C₃-C₈)-cycloalkyl, where in the alkyl groups in each case one to seven hydrogen atoms may be replaced by fluorine;
and their physiologically acceptable salts for preparing a medicament for reducing weight in mammals.

2. The use as claimed in claim 1 for the preparation of a medicament for the prophylaxis or treatment of obesity.

3. The use as claimed in claim 1 in combination with one or more active compounds suitable for reducing weight, for the preparation of a medicament for reducing weight in mammals.

4. The use as claimed in claim 1 in combination with one or more active compounds suitable for reducing weight, for the preparation of a medicament for the prophylaxis or treatment of obesity.

5. The use as claimed in claim 1 in combination with cathine, phenylpropanolamine, amfepramone, mefenorex, ephedrine, leptin, dexamphetamine, amphetamine, fenfluramine, dexfenfluramine, sibutramine, orlistat, mazindol or phentermine or their salts for preparing a medicament for reducing weight in mammals.

6. The use as claimed in claim 1 in combination with cathine, phenylpropanolamine, amfepramone, mefenorex, ephedrine, leptin, dexamphetamine, amphetamine, fenfluramine, dexfenfluramine, sibutramine, orlistat, mazindol or phentermine or their salts for preparing a medicament for the prophylaxis or treatment of obesity.

## Revendications

1. Utilisation des composés de formule I 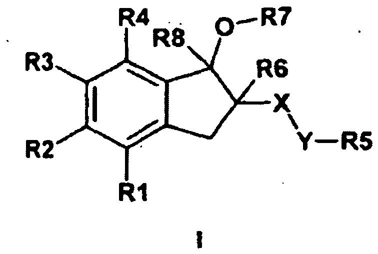 dans lesquels
R1, R2, R3, R4 signifient indépendamment les uns des autres H, F, Cl, Br, I, CN, N₃, NO₂, OH, O-alkyle en (C₁-C₈), O-cycloalkyle en (C₃-C₈), O-CH₂-phényle, O-phényle, O-CO-alkyle en (C₁-C₈), O-CO-cycloalkyle en (C₃-C₈), jusqu'à sept atomes d'hydrogène dans les radicaux alkyle pouvant être remplacés par fluor ;
S(O)₀₋₂-alkyle en (C₁-C₈), S(O)₀₋₂-cycloalkyle en (C₃-C₈), jusqu'à sept atomes d'hydrogène dans les radicaux alkyle pouvant être remplacés par fluor ; NH₂, NH-alkyle en (C₁-C₈), NH-cycloalkyle en (C₃-C₈), N[alkyle en (C₁-C₈)]₂, N[cycloalkyle en (C₃-C₈)]₂, NH-CO-alkyle en (C₁-C₈), NH-CO-cycloalkyle en (C₃-C₈) ;
S0₃H ; SO₂-NH₂, SO₂-NH-alkyle en (C₁-C₈), SO₂-NH-cycloalkyle en (C₃-C₈) ; SO₂-alkyle en (C₁-C₆) ;
NH-SO₂-NH₂ ; NH-SO₂-alkyle en (C₁-C₈), NH-SO₂-cycloalkyle en (C₃-C₈) ; O-CH₂-COOH, O-CH₂-CO-O-alkyle en (C₁-C₈), COOH, COO-alkyle en (C₁-C₈), CO-O-cycloalkyle en (C₃-C₈), CO-NH₂, CO-NH-alkyle en (C₁-C₈), CO-N[alkyle en (C₁-C₈)]₂ ;
alkyle en (C₁-C₈), cycloalkyle en (C₃-C₈), alcényle en (C₂-C₈), alcynyle en (C₂-C₈), un à sept atomes d'hydrogène dans les groupes alkyle, alcényle et alcynyle pouvant être remplacés par fluor ;
ou un hydrogène pouvant être remplacé par OH, OC(O)CH₃, O-CH₂-Ph, NH₂, NH-CO-CH₃ ou N(COOCH₂Ph)₂; phényle, 1- ou 2-naphtyle,
5-tétrazolyle, 1-[alkyle en (C₁-C₆)]-5-tétrazolyle, 2-[alkyle en (C₁-C₆)]-5-tétrazolyle ; 1-imidazolyle ;
1- ou 4-[1,2,4]-triazolyle,
2- ou 3-thiényle,
2- ou 3-furyle,
2-, 3- ou 4-pyridyle,
2-, 4- ou 5-oxazolyle,
3-, 4- ou 5-isoxazolyle,
2-, 4- ou 5-thiazolyle,
3-, 4- ou 5-isothiazolyle,
le radical aryle ou l'hétérocycle pouvant être substitué jusqu'à deux fois avec F, Cl, Br, CN,
OH, alkyle en (C₁-C₄), CF₃, O-alkyle en (C₁-C₄), S(O)₀₋₂-alkyle en (C₁-C₆), NH₂, NH-SO₂-alkyle en (C₁-C₄) ;
COOH, CO-O-alkyle en (C₁-C₄), CO-NH₂, et un à sept atomes d'hydrogène dans les groupes alkyle pouvant être remplacés par fluor ; ou
R2 et R3 forment ensemble le radical -O-CH₂-O- ;
X signifie S, SO, SO₂ ;
Y signifie (CH₂)ₚ, p pouvant valoir 0, 1, 2 ou 3 ;
R5 signifie alkyle en (C₁-C₁₈), cycloalkyle en (C₃-C₈), jusqu'à sept atomes d'hydrogène dans les groupes alkyle pouvant être remplacés par fluor ; (CH₂)₁₋₆-COOH, (CH₂)₁₋₆-COO-alkyle en (C₁-C₆), (CH₂)₁-₆-CONH₂ ;
CH₂-CH(NHR10)-COR11, R10 représentant H ou C(O)-alkyle en (C₁-C₆) et R11 représentant OH, O-alkyle en (C₁-C₆) ou NH₂ ;
phényle, 1- ou 2-naphtyle, biphényle ou un radical hétérocyclique, les cycles ou systèmes cycliques étant à chaque fois substitués jusqu'à trois fois avec
F, Cl, Br, I, CN, OH, O-alkyle en (C₁-C₈), 0-cycloalkyle en (C₃-C₈), O-CO-alkyle en (C₁-C₈), 0-CO-cycloalkyle en (C₃-C₈). S(O)₀₋₂-alkyle en (C₁-C₈), S(O)₀₋₂-cycloalkyle en (C₃-C₈), NH₂, NH-alkyle en (C₁-C₈), NH-cycloalkyle en (C₃-C₈), N[alkyle en (C₁-C₈)]₂, N[cycloalkyle en (C₃-C₈)]₂, NH-CO-alkyle en (C₁-C₈), NH-CO-cycloalkyle en (C₃-C₈), SO₃H ; SO₂-NH₂, SO₂-NH-alkyle en (C₁-C₈), SO₂-NH-cycloalkyle en (C₃-C₈), NH-SO₂-NH₂ ; NH-SO₂-alkyle en (C₁-C₈), NH-SO₂-cycloalkyle en (C₃-C₈); O-CH₂-COOH, O-CH₂-CO-O-alkyle en (C₁-C₈), COOH, CO-O-alkyle en (C₁-C₈), CO-O-cycloalkyle en (C₃-C₈), CO-NH₂, CO-NH-alkyle en (C₁-C₈), CO-N[alkyle en (C₁-C₈)]₂ ;
alkyle en (C₁-C₈), cycloalkyle en (C₃-C₈), un à sept atomes d'hydrogène dans les groupes alkyle pouvant à chaque fois être remplacés par fluor ;
R6 signifie (CH₂)₀₋₆-R9, (CH₂)₀₋₆-COOH, (CH₂)₀₋₆-COO-alkyle en (C₁-C₆), (CH₂)₀₋₆-CONH₂, (CH₂)₀₋₆-CH(NHR15)-COR16, F, Cl, Br, CN, alkyle en (C₁-C₁₈), cycloalkyle en (C₃-C₄), cycloalkyle en (C₆-C₈), jusqu'à sept atomes d'hydrogène dans les radicaux alkyle ou les radicaux cycloalkyle pouvant être remplacés par fluor ;
R15 signifie H, C(O)-alkyle en (C₁-C₆) ;
R16 signifie OH, O-alkyle en (C₁-C₆), NH₂ ;
R7 signifie (CH₂)₀₋₄R12, H, alkyle en (C₁-C₁₂), cycloalkyle en (C₃-C₄), cycloalkyle en (C₆-C₈), COO-alkyle en (C₁-C₆), COO-cycloalkyle en (C₃-C₈), jusqu'à sept atomes d'hydrogène dans les radicaux alkyle ou les radicaux cycloalkyle pouvant être remplacés par fluor ;
R8 signifie (CH₂)₀₋₄R14, alkyle en (C₁-C₁₂), cycloalkyle en (C₃-C₄), cycloalkyle en (C₆-C₈), jusqu'à sept atomes d'hydrogène dans les radicaux alkyle ou cycloalkyle pouvant être remplacés par des atomes de fluor ;
R9, R12, R14 signifient indépendamment les uns des autres phényle, 1- ou 2-naphtyle, biphényle ou un radical hétérocyclique, les cycles ou systèmes cycliques étant à chaque fois substitués jusqu'à trois fois avec
F, Cl, Br, I, CN, OH, O-alkyle en (C₁-C₈), O-cycloalkyle en (C₃-C₈), O-CO-alkyle en (C₁-C₈), O-CO-cycloalkyle en (C₃-C₈), S(O)₀₋₂-alkyle en (C₁-C₈), S(O)₀₋₂-cycloalkyle en (C₃-C₈), NH₂, NH-alkyle en (C₁-C₈), NH-cycloalkyle en (C₃-C₈), N[alkyle en (C₁-C₈)]₂, N[cycloalkyle en (C₃-C₈)]₂, NH-CO-alkyle en (C₁-C₈), NH-CO-cycloalkyle en (C₃-C₈), SO₃H ; SO₂-NH₂, SO₂-NH-alkyle en (C₁-C₈), SO₂-NH-cycloalkyle en (C₃-C₈), NH-SO₂-NH₂ ; NH-SO₂-alkyle en (C₁-C₈), NH-SO₂-cycloalkyle en (C₃-C₈); O-CH₂-COOH, O-CH₂-CO-O-alkyle en (C₁-C₈), COOH, CO-O-alkyle en (C₁-C₈), CO-O-cycloalkyle en (C₃-C₈), CO-NH₂, CO-NH-alkyle en (C₁-C₈), CO-N[alkyle en (C₁-C₈)]₂ ;
alkyle en (C₁-C₈), cycloalkyle en (C₃-C₈), un à sept atomes d'hydrogène dans les groupes alkyle pouvant à chaque fois être remplacés par fluor ;
ainsi que leurs sels physiologiquement compatibles, pour la fabrication d'un médicament pour la réduction du poids de mammifères.

2. Utilisation selon la revendication 1 pour la fabrication d'un médicament pour la prophylaxie ou le traitement de l'obésité.

3. Utilisation selon la revendication 1 en combinaison avec un ou plusieurs agents actifs réduisant le poids pour la fabrication d'un médicament pour la réduction du poids de mammifères.

4. Utilisation selon la revendication 1 en combinaison avec un ou plusieurs agents actifs réduisant le poids pour la fabrication d'un médicament pour la prophylaxie ou le traitement de l'obésité.

5. Utilisation selon la revendication 1 en combinaison avec des cathines, des phénylpropanolamines, des amfépramones, du méfénorex, des éphédrines, de la leptine, des dexamphétamines, des amphétamines, des fenfluramines, des dexfenfluramines, des sibutramines, de l'orlistat, du mazindol ou des phentermines ou leurs sels pour la fabrication d'un médicament pour la réduction du poids de mammifères.

6. Utilisation selon la revendication 1 en combinaison avec des cathines, des phénylpropanolamines, de l'amfépramone, du méfénorex, des éphédrines, de la leptine, des dexamphétamines, des amphétamines, des fenfluramines, des dexfenfluramines, des sibutramines, de l'orlistat, du mazindol ou des phentermines ou leurs sels pour la fabrication d'un médicament pour la prophylaxie ou le traitement de l'obésité.
